# EUROPEAN PATENT SPECIFICATION

(11) **EP 4 086 633 B1**
(45) Date of publication and mention of the grant of the patent: **13.08.2025**
(21) Application number: 22171247.4
(22) Date of filing: 03.05.2022
(51) Int. Cl.: G01N 33/68

(54) **PROGNOSTIC METHOD**
PROGNOSEVERFAHREN
PROCÉDÉ DE PRONOSTIC

(30) Priority: 04.05.2021 IT 202100011327
(43) Date of publication of application: 09.11.2022
(73) Proprietor: GEK S.r.l., 20146 Milano (IT)
(72) Inventor: BASELLO, Katia, 20146 Milano (IT); COSTANZI, Andrea, 20146 Milano (IT); SPECIANI, Attilio Francesco, 20146 Milano (IT); FIORENTIN, Enrico, 20146 Milano (IT); PIROLO, Marco, 20146 Milano (IT); SPECIANI, Michela Carola, 20146 Milano (IT); CAPPELLETTI, Mattia, 20146 Milano (IT)
(74) Representative: Rigamonti, Dorotea

(56) References cited:
- WO-A1-2014/123749
- WO-A1-2019/016156
- WO-A1-2019/130176
- WO-A1-2021/009182
- WO-A2-2008/061978
- WO-A2-2017/027611
- YASMINE ZOUGGARI ET AL: "B lymphocytes trigger monocyte mobilization and impair heart function after acute myocardial infarction", NATURE MEDICINE, vol. 19, no. 10, 15 September 2013 (2013-09-15), New York, pages 1273 - 1280, XP055473276, ISSN: 1078-8956, DOI: 10.1038/nm.3284

## Description

The present invention relates to a method for assessing the response of a subject who has undergone an ischemic event to a pharmacological treatment.

### BACKGROUND ART

The high incidence of cardiovascular diseases in the population and the availability of drugs for managing the phenomena that follow, has pushed the research towards the identification of markers which could predict the effectiveness thereof. Said need is particularly felt where the drugs available are particularly expensive drugs, for example monoclonal antibodies, and the effect of which is highly subjective.

WO2008/061978 describes a method for classifying subjects who have undergone cardiovascular events in order to select the appropriate pharmacological treatment, where said method comprises measuring Troponin levels in a biological fluid.

Zouggari Y et al., in Nat. Med. 2013, 19: 1273-1280, describe that high circulating levels of BAFF following myocardial infarction are predictive of an increased risk of subsequent cardiac events.

WO2019/130176 also correlates high BAFF levels as predictive of post-infarction complications.

WO2017/130176 describes a method for defining a therapeutic approach in diabetic subjects, without defining a patient subgroup that has previously a cardiovascular event.

WO2014/123749 describes treatment of diabetic patients having complications such as CVD, characterized by MGO overexpression. WO2021/009182 describes a correlation between MGO and diabetes. The need for a method for classifying subjects following cardiovascular episodes for the selection of the most suitable therapeutic approach for the specific case remains strongly felt.

Blood tests are generally carried out from a sample of liquid blood, serum or plasma, stored in appropriate tubes which may or may not contain an anticoagulant according to the type of analysis to be performed. There is a strong need for a method adapted to make a blood sample available for subsequent analysis, which overcomes the limits of storage, transport and risks of infection deriving from contaminated samples, typically associated with liquid blood samples.

In a further aspect, the need is to have immediate analyses of said sample.

### DESCRIPTION OF THE INVENTION

### Description of the drawings

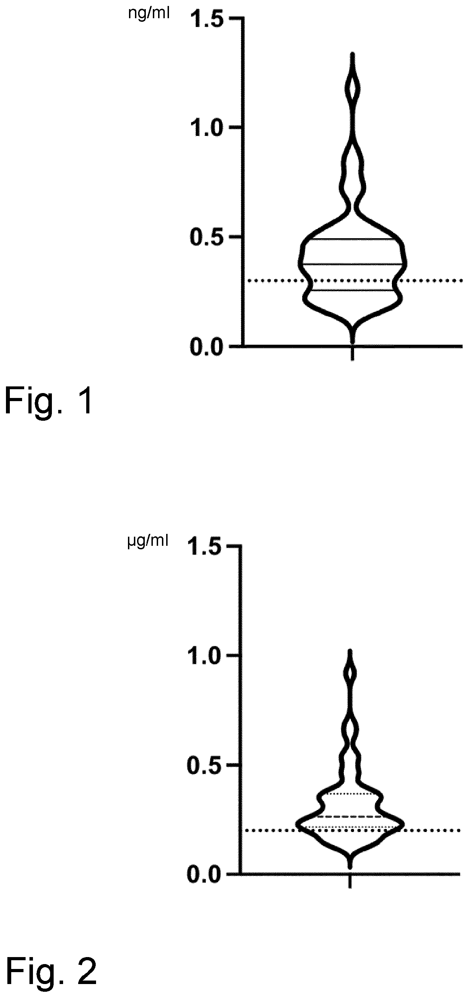

**Figure 1****:** (description purpose only) violin plot showing the distribution of BAFF values measured in the cohort. The BAFF concentration (ng/ml) is expressed in ordinate. The width of the violin is indicative of the number of the measured value. The lines identify the median, 25th, and 75th percentiles. The dotted line is the cut-off according to the invention.
**Figure 2****:** violin plot showing the distribution of MGO values measured in the cohort. The MGO concentration (µg/ml) is expressed in ordinate. The width of the violin is indicative of the number of the measured value. The lines identify the median, 25th, and 75th percentiles. The dotted line is the cut-off according to the invention.

The present invention relates to an in vitro method for predicting the pharmacological response in subjects who have undergone a cardiac ischemic event (first event or subsequent to the first). It is here described a method that comprises determining the expression levels of BAFF and/or Methylglyoxal, and optionally further markers, in a biological fluid sample of a subject. It is here claimed a method that comprises determining the expression levels of Methylglyoxal, and optionally further markers, in a biological fluid sample of a subject.

Said biological fluid is selected from the group comprising saliva, urine, blood. In an embodiment, it is blood.

In a preferred embodiment, said determination is carried out on dried blood.

For description purpose only, the authors of the present invention have surprisingly demonstrated the possibility of selecting among the subjects in whom a cardiac episode occurred, a subpopulation eligible for treatment with antiCD20, where said subpopulation corresponds to the subset of infarcted subjects which shows higher BAFF values than the mean value measured in the cohort of subjects following a cardiac episode, defined as the BAFF subset.

In a preferred form, said measurement occurs acutely, when deciding on the optimal reperfusion strategy.

In a preferred form, BAFF values measured in dried blood in excess of 0.300 ng/ml are classified in said BAFF subset.

The authors of the present invention have surprisingly demonstrated the possibility of selecting, among the subjects in whom a cardiac episode occurred, a subpopulation eligible for treatment with antidiabetics, where said subpopulation corresponds to the subset of infarcted subjects showing higher values of MGO bound to the amino acids of proteins than the mean value measured in the cohort of subjects following a cardiac episode, defined as the MGO subset.

In a preferred form, said measurement occurs acutely, when deciding on the optimal reperfusion strategy.

In a preferred form, MGO values measured in dried blood in excess of 0.200 µg/ml are classified in said MGO subset.

It is here described a method for defining a therapeutic approach in subjects following a cardiovascular episode is claimed here, comprising:
- Measuring, in a biological fluid obtained from a subject following a cardiovascular episode, the levels of at least one marker selected from the group comprising BAFF, MGO bound to the amino acids of proteins;
- Where said method comprises creating at least one subset, said subsets being:
   ∘ Subset defined as BAFF, to which subjects in whom the BAFF levels are higher than 0.300 ng/ml are assigned;
   ∘ Subset defined as MGO, to which subjects in whom the MGO levels are higher than 0.200 µg/ml are assigned;
- Where said one or more subsets are proper or improper subsets;
- Where said subsets, if at least two, are superimposed or disjointed;
- Said BAFF subset corresponds to the subpopulation eligible for treatment with antiCD20;
- Said MGO subset corresponds to the subpopulation eligible for treatment with antidiabetics.

It is here claimed a method for defining a therapeutic approach in subjects following a cardiovascular episode is claimed here, comprising:
- Measuring, in a biological fluid obtained from a subject following a cardiovascular episode, the levels of at least MGO bound to the amino acids of proteins;
- Where said method comprises creating at least one subset, said subsets being:
   ∘ Subset defined as MGO, to which subjects in whom the MGO levels are higher than 0.200 µg/ml are assigned;
- Where said one or more subsets are proper or improper subsets;
- Where said subsets, if at least two, are superimposed or disjointed;
- Said MGO subset corresponds to the subpopulation eligible for treatment with antidiabetics.

In a preferred form, said biological fluid is dried blood.

In a preferred form, said measurement is carried out by immunochemical assay, for example by enzyme immunoassay (ELISA assay) or by immunochromatography, for example by card immunochromatography (lateral flow), with optical or fluorometric reading.

In a further embodiment, said method comprises measuring further markers, so as to obtain further subsets.

By way of example, those skilled in the art know that the presence of MRP4 is indicative of a lack of responsiveness to treatment with salicylates (Floyd CN. Pharmacol Ther 2014;141:69-78). Therefore, the method according to the present invention may further comprise measuring MRP4, comprising the creation of a further subset, referred to as the MRP4 subset, to which subjects in whom the MRP4 levels are equal to 0 are assigned. Said subset comprises the subjects responsive to treatment with salicylates.

Therefore, those skilled in the art know the drug to be administered to subjects not belonging to said MRP4 subset, for example an MRP4 antagonist molecule or anticoagulants and/or antiplatelets other than salicylates.

The present disclosure (not claimed) further relates to a method for the remote collection of a biological fluid and subsequent laboratory analysis of at least one analyte contained in said fluid, where said method, the dried blood method, comprises:
a) Providing a support for the collection of a biological fluid, where said support is made of a synthetic material;
b) Soaking said support with a biological fluid of interest;
c) Transporting and/or storing said support until the time of analysis;
d) Eluting said sample;
e) Analyzing the eluted material.

The measurements carried out on dried blood are measurements carried out on a material obtained according to the above method.

The following examples have the sole purpose of better illustrating the invention and are not to be intended as limiting it in any manner, the scope of which is defined by the claims.

### Example 1: Defining the cohort and obtaining blood samples

A cohort of 38 subjects following infarction was randomly selected, homogeneous by Caucasian ethnicity and with age and co-morbidity features representative of the population at higher cardiovascular risk. From said subjects, an arterial blood sample was obtained during the reperfusion procedure. Said blood was collected and stored on synthetic swab according to the dried blood method.

### Example 2: (for description purpose only) BAFF subset

The BAFF values were measured on the samples obtained and stored according to example 1.

The pre-analytical extraction from dried blood synthetic swab occurs with the following protocol: Copan 552C swab is reconstituted with 150 µl PBS and vortexed for 10 seconds. The liquid sample is transferred to a new 1.5 ml tube, centrifuged for 8 minutes at 3200 rpm at 4°C. The supernatant is transferred to a new 1.5 ml tube and immediately frozen at -20°C. The sample is then thawed at room temperature and loaded onto the specific ELISA plate, according to the instructions of the ELISA protocol indicated by the manufacturer. The ELISA kits used are based on the sandwich methodology including the use of pairs of mono- and/or polyclonal antibodies. In particular, the BAFF levels were measured with specific antibodies using the commercial kit hBAFF ELISA R&D Systems (Minneapolis, USA).

The data obtained, shown in figure 1, show the values obtained in ordinate, expressed in ng/ml. The data are distributed between the values of 0.177 and 1.18 ng/ml. The median is 0.375 ng/ml, the 25th percentile 0.257 ng/ml, and the 75th percentile 0.491.

Said cohort thus identifies a subset, defined as the BAFF subset. Said subset includes subjects with BAFF values in excess of 0.300 ng/ml. Said value is indicated by the dotted line in figure 1.

68% of the tested cohort falls in said subset.

### Example 3: MGO subset (according to the invention)

On the samples obtained and stored according to example 1, the values of MGO bound to the amino acids of proteins were measured. At the time of analysis, the sample was eluted from the synthetic swab, following the procedure described for example 2.

MGO was measured with OxySelect^{™} Methylglyoxal Competitive ELISA Kit - Cell Biolabs, San Diego, CA, USA, an enzyme immunoassay developed for the detection and quantification of MGO-H1 (methylglyoxal-hydro-imidazolone) protein adducts. An anti-MgO antibody coats the surface of the well of the ELISA plate. The samples containing the standard MGO or MGO-BSA protein adducts are added to the preabsorbed plate of the MGO conjugate. After a short incubation, an anti-MgO antibody is added, followed by a secondary antibody conjugated with HRP. The content of the MGO protein adducts in the unknown samples are determined by comparing the absorbances of the samples with those of the standard MGO-BSA curve at known concentration.

The data obtained, shown in figure 2, show the values obtained in ordinate, expressed in µg/ml. The data are distributed between the values 0.135 and 0.919 µg/ml. The median is 0.264, the 25th percentile 0.217, and the 75th percentile 0.369 µg /ml.

Said cohort thus identifies a subset, defined as the MGO subset. Said subset includes subjects with MGO values in excess of 0.200 µg /ml. Said value is indicated by the dotted line in figure 2.

81% of the tested cohort falls in said subset.

## Claims

1. A method for defining a therapeutic approach in subjects following a cardiovascular event, said method comprising:
- measuring, in a biological fluid previously provided from a subject, the levels of at least one marker which is MGO (Methylglyoxal) bound to the amino acids of proteins;
- wherein said method comprises creating at least one subset, said subsets being:
∘ Subset defined as MGO, to which subjects in whom the MGO levels are higher than 0.200 µg/ml are assigned;
- where said one or more subsets are proper or improper subsets;
- where said subsets, if at least two, are superimposed or disjointed;
- said MGO subset corresponds to the subpopulation eligible for treatment with antidiabetics.

2. The method according to claim 1, further comprising:
- measuring, in said fluid, the levels of BAFF (B-Cell Activating Factor);
- wherein said method comprises creating an additional subset, said additional subset being:
∘ Subset defined as BAFF, to which subjects in whom the BAFF levels are higher than 0.300 ng/ml are assigned;
- where said BAFF subset corresponds to the subpopulation eligible for treatment with antiCD20.

3. A method according to claim 1 or 2 which further comprises measuring further markers, so as to obtain further subsets.

4. A method according to claims 1 to 3, wherein one of said further markers is MRP4, and subjects in whom the MRP4 levels are equal to 0 are assigned to said MRP4 subset and said MRP4 subset corresponds to the subpopulation responsive to treatment with salicylates.

5. A method according to one of claims 1 to 4, wherein said biological fluid is arterial, venous, or dried blood.

6. A method according to one of claims 1 to 5, wherein said levels are measured by immunochemical assay, for example by enzyme immunoassay (ELISA assay), or by immunochromatography, for example by card immunochromatography (lateral flow), with optical or fluorometric reading.

## Patentansprüche

1. Verfahren zum Definieren eines therapeutischen Ansatzes bei Probanden nach einem kardiovaskulären Ereignis, das genannte Verfahren umfassend:
- Messen, in einer biologischen Flüssigkeit, welche zuvor von einem Probanden bereitgestellt wurde, der Spiegel von mindestens einem Marker, welcher an die Aminosäuren von Proteinen gebundenes MGO (Methylglyoxal) ist;
- wobei das genannte Verfahren das Erzeugen mindestens einer Teilmenge umfasst, wobei diese Teilmengen sind:
∘ als MGO definierte Teilmenge, welcher Probanden zugeordnet werden, deren MGO-Spiegel höher als 0,200 µg/ml ist;
- wobei die genannte eine oder die mehreren Teilmengen echte oder unechte Teilmengen sind;
- wobei diese genannten Teilmengen, soweit sie mindestens zwei sind, sich überschneiden oder nicht zusammenhängen;
- die genannte MGO-Teilmenge entspricht der für eine Behandlung mit Antidiabetika geeigneten Teilpopulation.

2. Verfahren nach Anspruch 1, weiterhin umfassend:
- Messen, in der genannten Flüssigkeit, des BAFF-Spiegels (B-Zell-aktivierender Faktor);
- wobei das genannte Verfahren das Erzeugen einer zusätzlichen Teilmenge umfasst, wobei die genannte zusätzliche Teilmenge ist:
∘ als BAFF definierte Teilmenge, der Probanden mit BAFF-Werten über 0,300 ng/ml zugeordnet werden;
- wobei die BAFF-Teilmenge der Teilpopulation entspricht, welche für eine Behandlung mit AntiCD20 geeignet ist.

3. Verfahren nach Anspruch 1 oder 2, ferner umfassend das Messen von weiteren Markern, um weitere Teilmengen zu erhalten.

4. Verfahren nach einem der Ansprüche 1 bis 3, wobei einer der genannten weiteren Marker MRP4 ist und Probanden, deren MRP4-Spiegel gleich 0 sind, der genannten MRP4-Teilmenge zugeordnet werden und die genannte MRP4-Teilmenge der auf die Behandlung mit Salicylaten ansprechenden Teilpopulation entspricht.

5. Verfahren nach einem der Ansprüche 1 bis 4, wobei die genannte biologische Flüssigkeit arterielles Blut, Venenblut oder Trockenblut ist.

6. Verfahren nach einem der Ansprüche 1 bis 5, **dadurch gekennzeichnet, dass** die genannten Spiegel durch einen immunchemischen Assay, z.B. durch einen Enzymimmunoassay (ELISA-Assay), oder durch Immunochromatographie, z.B. durch Teststreifen-Immunochromatographie (seitlicher Flusstest), mit optischer oder fluorometrischer Ablesung gemessen werden.

## Revendications

1. - Procédé pour définir une approche thérapeutique chez des sujets après un événement cardiovasculaire, ledit procédé comprenant :
- mesurer, dans un fluide biologique préalablement fourni par un sujet, les taux d'au moins un marqueur qui est le MGO (Méthylglyoxal) lié aux acides aminés de protéines ;
- ledit procédé comprend la création d'au moins un sous-ensemble, lesdits sous-ensembles étant :
∘ Sous-ensemble défini comme MGO, auquel sont attribués des sujets dont les taux de MGO sont supérieurs à 0,200 µg/ml ;
- où ledit ou lesdits sous-ensembles sont des sous-ensembles appropriés ou inappropriés ;
- où lesdits sous-ensembles, s'il y en a au moins deux, sont superposés ou disjoints ;
- ledit sous-ensemble MGO correspond à la sous-population éligible au traitement par antidiabétique.

2. - Procédé selon la revendication 1, comprenant en outre :
- mesurer, dans ledit fluide, les taux de BAFF (Facteur d'Activation des Lymphocytes B) ;
- ledit procédé comprend la création d'un sous-ensemble supplémentaire, ledit sous-ensemble supplémentaire étant :
∘ Sous-ensemble défini comme BAFF, auquel sont attribués des sujets dont les taux de BAFF sont supérieurs à 0,300 ng/ml ;
- où ledit sous-ensemble BAFF correspond à la sous-population éligible au traitement par antiCD20.

3. - Procédé selon l'une des revendications 1 ou 2 qui comprend en outre la mesure d'autres marqueurs, afin d'obtenir d'autres sous-ensembles.

4. - Procédé selon l'une des revendications 1 à 3, dans lequel l'un desdits autres marqueurs est MRP4, et les sujets chez qui les taux de MRP4 sont égaux à 0 sont attribués audit sous-ensemble MRP4 et ledit sous-ensemble MRP4 correspond à la sous-population sensible au traitement par des salicylates.

5. - Procédé selon l'une des revendications 1 à 4, dans lequel le fluide biologique est du sang artériel, veineux ou séché.

6. - Procédé selon l'une des revendications 1 à 5, dans lequel lesdits taux sont mesurés par dosage immunochimique, par exemple par dosage immunoenzymatique (dosage ELISA), ou par immunochromatographie, par exemple par immunochromatographie sur carte (flux latéral), avec lecture optique ou fluorométrique.
